# EUROPEAN PATENT SPECIFICATION

(11) **EP 1 318 848 B1**
(45) Date of publication and mention of the grant of the patent: **30.07.2008**
(21) Application number: 01971489.8
(22) Date of filing: 21.09.2001
(51) Int. Cl.: A61M 1/12

(54) **HEART ASSIST DEVICE**
HERZUNTERSTÜTZUNGSVORRICHTUNG
DISPOSITIF D'ASSISTANCE CARDIAQUE

(30) Priority: 22.09.2000 AU PR031200
(43) Date of publication of application: 18.06.2003
(73) Proprietor: Sunshine Heart Company Pty Ltd., Birchgrove, NSW 2041 (AU)
(72) Inventor: PETERS, William, Suttle, Ackland 1005 (NZ); MARSH, Peter, Crispin, Lawrence, Birchgrove, NSW 2041 (AU); WHITE, Geoffrey, Hamilton, East Balmain, NSW 2041 (AU); HENRICHSEN, Hans, Hansforth, Wollongon, NSW 2500 (AU); SNOW, David, Bedford, NH 03110 (US)
(74) Representative: Smith, Norman Ian
(86) International application number: PCT/AU2001/001187
(87) International publication number: WO 2002/024255

(56) References cited:
- WO-A-92/08500
- WO-A-98/05289
- WO-A2-00/76288
- US-A- 4 583 523
- US-A- 4 809 676
- US-A- 4 979 936

## Description

### FIELD OF THE INVENTION

The present invention relates generally to counterpulsation heart assist devices and systems.

### BACKGROUND OF THE INVENTION

The intra-aortic balloon pump (IABP) was first proposed in the 1960's as a method of partial support for an acutely failing heart, for example, after heart surgery or heart attack. An IABP comprises a long thin catheter [7-12 Fr] with an elongated balloon at its tip [volume 25-50ml]. The balloon is inserted via the femoral artery and inflated and deflated in counter-pulsation with the heart beat. Inflation in diastole causes a diastolic pressure pulse with increase in blood flow to the relaxing heart muscle. Deflation of the balloon immediately before the heart ejects (presystole) reduces the pressure head against which the left ventricle has to eject blood, improving cardiac output and reducing the work of the heart. Early investigators determined that the best and most efficient balloon position was closest to the heart, i.e., in the ascending aorta. However, in recent times, the balloon is positioned via the femoral artery in the descending aorta for short term (1-10 days) use.

IABP counterpulsation has been proven to work very well in the short-term to assist hearts to recover when drugs (ionotropes etc.) are insufficient or inappropriate to support the cardiovascular system.

IABP's operating in counterpulsation assist the heart function. When inflated, the balloon propels blood peripherally from within the aorta to improve blood circulation in the patient. Moreover, more blood is forced into the coronary arteries to help nourish and strengthen the heart muscle. However, disadvantages of IABPs that limit their application are: the patient is bedridden and not able to sit or walk; there is very high incidence of limb ischemia and complications; and the IABP are not able to be left in for any period of time greater than 10 days. A further disadvantage is the balloon comes into direct contact with the blood flowing into the aorta, which can cause damage to the blood cells and a risk of thromboembolism. In addition, current IABP systems are inflated by means of a tube passing through the body and directly into a groin vessel, the femoral artery, which results in a high risk of associated leg complications. Additionally, the use of a gas to inflate the balloon is not an entirely safe operation since any leakage of gas from the balloon into the blood stream could cause a gas embolus.

Aortic compression (periaortic diastolic compression) has also been described as a means to increase coronary blood flow, e.g. US Patent No. 4,583,523 and US Patent No. 4,979,936. The Applicant's international PCT patent application No. PCY/AU00/00654 (international publication No. WO 00/76288) entitled "Heart Assist Devices Systems and Methods" discloses heart assist devices that can be quickly and totally implanted in a relatively easy manner and with minimum trauma to a patient The disclosed devices advantageously have no blood contacting surfaces and do not require cardiopulmonary bypass for implantation. Generally speaking, the devices disclosed include and aortic compression means adapted, when actuated, to compress an aorta, a fluid reservoir and a pump means adapted to pump a fluid fro the fluid reservoir to the aortic compression means to actuate same. The fluid reservoir is adapted to be wholly positioned within the chest cavity of the patient

WO98/05289 describes a device for performing direct cardiac massage including an inflatable bladder mounted on a rigid inflation tube. After insertion, the bladder is repeatedly inflated and deflated to massage the heart and provide blood flow.

It would be desirable to have a heart assist device that could be quickly implanted and explanted in a relatively easy manner and with minimum trauma to the patient and to allow ambulation with low risk of complications.

### SUMMARY OF THE INVENTION

Accordingly, in a first aspect, the present invention provides an implantable device for assisting the functioning of the heat of a patient according to claim 1.

In a second aspect, the present invention provides a device for assisting the functioning of the hear of a patient according to claim 2.

Minimally invasive surgery preferably includes endoscopic surgery.

Preferably, the releasing means is adapted to allow minimally invasive surgical or non-surgical removal of the device from the patient's thoracic cavity.

Alternatively, the releasing means is adapted to allow minimally invasive surgical or non-surgical de-activation of the device (to disable compressing of the aorta) and retention in the patient's thoracic cavity.

The heart assist device of the present invention allows, at least in preferred embodiments, partial unloading of the heart, augmenting of the cardiac output of the heart, increased coronary artery blood flow and substantial recovery of the heart so that the device could be weaned Further, the device advantageously has no blood contacting surfaces. Additionally, the device does not require cardiopulmonary bypass for implantation and can therefore be turned on and off without significant patient risk.

After use, it is preferable for the device to be released and removed from the body, advantageously without requiring further surgery. Alternatively, the device can be left in situ, in an inactive state, until needed again. Alternatively, the device can be left in situ for on-demand, spaced-apart sessions of counterpulsation for treatment or relief from chronic myocardial ischaemia and/or heart failure. The provision of releasing means ensures that however the device is used it may be readily removed at any time without difficulty.

An advantage of at the least the preferred embodiments of the device and system of the present invention is the risk of limb ischaemia associated with conventional IABP systems is avoided because there is no blood contact with the implanted device - particularly as there is no catheter placed in a small lumen vessel -vessel size is an independent risk factor for limb ischemia using IABP. Patient ambulation is also possible. Additionally, the implantation technique used for the device of the invention is less invasive than those required for other devices. In particular, compared to the arrangement taught in US Patent No. 4,583,523, the device of the present invention provides a better outcome in term of reduced risk of infection, cosmesis and ease of implant and explant. Another advantage of at the least the preferred embodiments of the device and system of the present invention is that there is little risk to the patient in the event of the device failure. A gas leak may cause a pneumothorax, but would not cause intravascular gas embolism as is known with IABPs. The preferred embodiments of device also have the significant advantage of being able to be weaned and turned off in the event of cardiac recovery. This is not possible with known implantable heart assist devices such as LVADs. If the heart shows signs of recovery, the device can be retrieved by remotely releasing the compressing means and withdrawing the device. However, if the heart shows signs of relapsing back into failure, the device can be switched back on.

The compressing means of the device of the present invention preferably includes an inflatable cuff or one or more preshaped balloons for positioning against or around a portion of the aorta or for wrapping around a portion of the aorta. Preferably, the balloon(s)/cuff is/are configured longitudinally to fit the curve of the ascending aorta. In a particularly preferred form of the device of the present invention, the cross-section of the balloon(s)/cuff is/are C-shaped, allowing wrapping of the balloon(s)/cuff, preferably with some overlap, around the aorta. In one embodiment of the invention the balloon(s)/cuff is/are shaped such that they concentrically compresses the length of enclosed aorta and spread the compression forces evenly, thereby reducing any wear or fatigue on any one part of the aorta. Alternatively, the balloon(s)/cuff can inflate assymetrically about the aorta. The sleeve may have an elongated "tongue" on one arm of the C-shaped balloon(s)/cuff that is passed around the aorta to be secured by suturing or other means on the outer aspect of the arm of the C-arm to which the wrap is secured, by sutures, staples, or the like. The release mechanism may be part of the securing means or separate from the securing means. Furthermore, the preshaped balloon(s)/cuff and flexible sleeve are particularly designed to create a snug fit and low profile on the aorta, to reduce damage to the aorta and surrounding structures, and to create maximum efficiency of the device.

The balloon(s)/cuff is/are preferably made from a very thin synthetic plastics material desirably having an inner wall that is elastic or inelastic. If the inner is inelastic, it is preferably so shaped that it can be moved inwardly as the balloon(s)/cuff is/are inflated. The outer wall of the balloon(s)/cuff is/are preferably inelastic. If the wrap extends around the whole balloon(s)/cuff the outer wall could be made of an elastic material. In a further alternative, outfolds or extensions of the inelastic outer wall are used to achieve the wrap without the need for a separate wrap material. In one preferred form, the elastic materials in which the balloon(s)/cuff is/are made include silicones. Relatively inelastic or inelastic plastics (at the operating pressures) in which the balloon(s)/cuff is/are made include polyurethanes, co-polymers of silicones and urethanes, PET and PTFE.

The balloon(s)/cuff are preferably connected to a catheter which extends out of the body and which is adapted for carrying the inflating fluid into and out of the balloon(s)/cuff. The fluid is preferably a gas, most preferably helium. The catheter is preferably also used to withdraw the balloon(s)/cuff from the patient and is connected to the balloon(s)/cuff sufficiently securely that the force of withdrawal will not detach the catheter from the balloon(s)/cuff.

In preferred forms, the wrap extends around the whole balloon(s)/cuff and is connected onto itself, or connected to the balloon(s)/cuff at each end, or extends only across the gap between the ends of the baUoon(s)/cuff. In the latter case, the wrap is connected at each end to, or integral with, the balloon(s)/cuff.

In other preferred forms, the wrap is separate from the balloon(s)/cuff, or integral with the balloon(s)/cuff.

The wrap is desirably as thin and flexible as possible but also sufficiently inelastic so as to not stretch when the balloon(s)/cuff is/are inflated. The wrap is preferably formed of a woven dacron material such as "Twillweave"(regd. Trade Mark) or from a sheet of synthetic plastics film. Suitable synthetic plastics include PET, PTFE and polyurethanes. If the wrap is formed of a woven dacron it is desirably coated in a smooth and low friction plastics material such as PTFE. In another form, the wrap is made of a memory shape plastic, and when it is desired to remove the wrap it is heated or cooled slightly to cause it to shrink into a more compact form.

The means to secure the ends of the wrap together can be the same means as are involved in the release of the wrap or may be separate from the release means. The securing means preferably comprises a row of suture stitches made by the surgeon when placing the device in the patient. These stitches being placed between the ends of the wrap or between one end of the wrap and a part of the balloon. Stitches allow the surgeon to easily place the cuff with the required tightness around the aorta, some of the other possible securement arrangements may not provide this flexibility. As an alternative to stitches, the securing means can be: an adhesive patch on the wrap which can stick onto a corresponding part of the wrap or the balloon(s)/cuff, the adhesive patch can optionally be covered with a "peel off' strip until used; a sliding clasp fastener; or one or more "bundle tie" type ratchet connectors. Alternatively, an end of the wrap can be sutured, or otherwise connected, to a release thread extending down the catheter, which thread is connected to the balloon(s)/cuff. The two ends of the wrap, or one end of the wrap and one part of the balloon(s)/cuff, being provided with hooks or holes through which a thread can be laced and drawn tight.

The release mechanism is preferably: a wire which extends down the catheter and may be pulled to release one end of the wrap; a thread extending down the catheter to which one end of the wrap is connected which can be released, an advantage of this arrangement is that the wrap can be tightened or loosened around the aorta during the operation or while it is in place on the patient post operation; a thread extending down the catheter and connected to a sliding clasp connector which can be pulled to release the sliding clasp connector; a thread extending down the catheter and connected to a knife blade positioned relative to sutures or other connecting means so that pulling of the thread causes the knife blade to sever the sutures; an electric wire embedded in the wrap so that passage of a brief electric current along the wire will fuse or melt the connection.

The release mechanism is preferably adapted such that, after release, the wrap is drawn into a tube adjacent to the catheter, thereby assisting the withdrawal of the device from the patient.

The placement of the device is typically during an upper mid-line sternotomy. The balloon(s)/cuff is placed around the aorta and the catheter placed to extend transcutaneously. The catheter can be extended downwardly from the aorta and emerge from the patient between the ribs or below the costal margin on the right or left hand side of the patient or extended upwardly to emerge in the region of the supra sternal notch. The device can also be placed thoracoscopically via thoracic ports in the right and/or left chest.

The removal of the device is effected by releasing the release mechanism and gently withdrawing the balloon(s)/cuff from the patient. If desired, a trocar, possibly with an expandable conical proximal end, is slid down the catheter to assist in the withdrawal of the balloon(s)/cuff through the skin. The trocar being slid down the catheter until it is inside the chest cavity, thereafter its conical end is expanded to form a funnel into which the balloon(s)/cuff is/are drawnIf present the conical end can then be collapsed to compress the cuff. It may be also desirable to actively deflate the balloon to reduce it to the smallest possible cross sectional size. The balloon(s)/cuff can also be formed with fluoroscopic markers so that the removal procedure may be performed under real time fluoroscopy. This alerts the surgeon to any problems which might cause him/her to leave the balloon(s)/cuff in place in the patient or to perform minor surgery to remove the cuff. The physical separation of the balloon(s)/cuff from the aorta and any tissue which has grown around the device is assisted by inflating the balloon(s)/cuff after the wrap has been released from around the aorta.

In a preferred form of the invention, the device is adapted for compression of the ascending aorta. An upper mid-line sternotomy provides easy surgical access to the ascending aorta and has the further advantage of not being very painful for the patient. A minimum incision is required in this procedure. alternatively the device may be placed by minimally invasive surgery between the ribs.

The cuff has a single inlet/outlet port for the passage of fluid to inflate/deflate the cuff. The fluid used is preferably gas, such as air or helium. The port and connecting tube to the motive means is of sufficient diameter and length to allow rapid emptying and filling of the cuff without generating too high compression pressures. In a preferred mode of use of the device of the invention, the compressing means is preferably adapted to squeeze approximately 15-25ml of blood form the ascending aorta in each compression cycle. The fluid preferably moves within 0.15sec for effective counterpulsation action. The compressive force emptying the cuff is the force exerted by the compressed aorta. This is approximately 100 mmHg. A tube lumen of approximately 0.5cm with a length of 20-30cm allows 25ml gas to pass down a gradient of 100mmHg in less than 0.15sec. The compressive force filling the cuff is generated by the motive means, and this pressure gradient is approximately the same (ie the motive means generates approximately 200 mmHg) to allow the fluid to shift into the cuff in less than 0.15 sec.

The port preferably has a trumpet-shaped or flanged opening into the cuff to spread the fluid more evenly into the cuff during inflation and to assist more rapid deflation. The trumpet shaped port also results in a tapered exterior surface of the cuff which facilitates removal of the device percutaneously via a small incision in the chest wall. A diffuser can also mount within the lumen of the port to reduce the fluid force on the balloon cuff during inflation.

The motive means of the device of the invention can be any means that is capable of cyclically compressing and decompressing the fluid sac. The motive means can include or be associated with means for detecting speed and completeness of cuff filling and emptying, and of monitoring the fluid pressure within the connector tube, means for measuring arterial blood pressure or flow. The motive means can also act to record the ECG.. An example of a suitable motive means is the Datascope 97 IABP console or the Arrow ACAT console. The cuff may also have holes or slits to accommodate coronary artery bypass grafts to the ascending aorta. Alternatively, grafts can be positioned distal or proximal to the cuff.

The device of the invention advantageously allows relief/recovery from heart failure whilst allowing the patient to move around freely, for unlimited periods of time and without the risk of limb complications while allowing the device to be removed easily should that de required or desired.

### BRIEF DESCRIPTION OF THE DRAWINGS

Preferred embodiments of the invention will now be described, by way of examples only, with reference to the accompanying drawings in which:
Fig. 1 is a perspective view of an implantable device which is helpful in understanding the invention positioned around the ascending aorta (release mechanism not shown);
Fig. 2 is a side view of the device shown in Fig. 1 (release mechanism not shown);
Fig. 3 is a cross-sectional view of a device similar to that shown in Fig. 2 along line 3-3.
Fig. 4 is a partial perspective view of a first embodiment of a release mechanism (in a close position) suitable for use in embodiments of implantable devices according to the invention;
Fig. 5 is a partial perspective view of the mechanism shown in Fig. 4 (in a released position);
Fig. 6 is a side view of a second embodiment of an implantable device according to the invention;
Fig. 7 is an end view of the device shown in Fig. 6;
Fig. 8 is a side view of a third embodiment of an implantable device according to the invention;
Fig. 9 is an end view of the device shown in Fig. 8;
Fig. 10 is a side view of a balloon used in the device shown in Fig. 8;
Fig. 11 is an end view of the balloon shown in Fig. 10;
Fig. 12 is a side view of a fourth embodiment of an implantable device according to the invention;
Fig. 13 is a further side view of the device shown in Fig. 12;
Fig. 14 is an opposite side view to that of Fig. 13 of the device shown in Fig. 12;
Fig. 15 is a side view of an embodiment of a balloon suitable for use in implantable devices according to the invention;
Fig. 16 is an end view of the device shown in Fig. 15;
Fig. 17 a side view of another embodiment of a balloon suitable for use in implantable devices according to the invention;
Fig. 18 is an end view of the device shown in Fig. 17;
Fig. 19 is a side view of yet another embodiment of a balloon suitable for use in implantable devices according to the invention;
Fig. 20 is an end view of the device shown in Fig. 19; and
Fig. 21 is a side view of a yet further embodiment of a balloon suitable for use in implantable devices according to the invention.

### DETAILED DESCRIPTION OF THE PREFERRED EMBODIMENTS

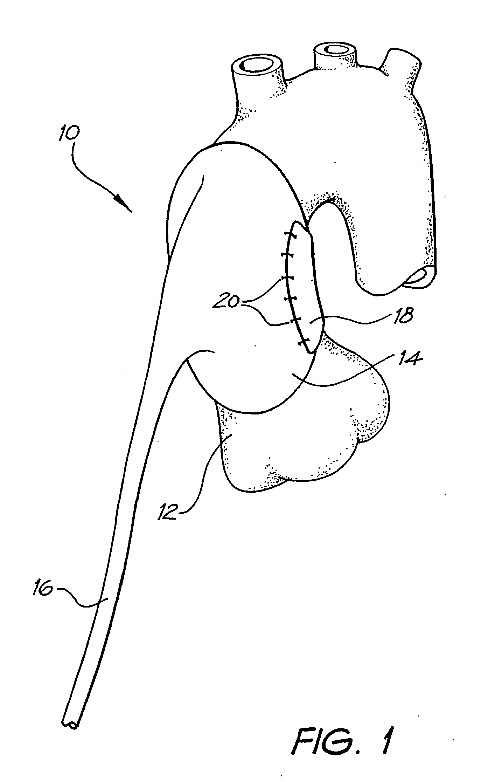

Fig. 1 shows a schematic perspective view of a heart assist device 10 helpful in understanding the invention. The device 10 is suitable for implantation in the thoracic cavity of a patent adjacent the ascending portion of the aorta 12, as shown. The device 10 includes a flexible hollow inflatable cuff 14 and a percutaneous gas line 16. The cuff 14 is curved along its length so as to substantially replicate the curve of the aorta 12 adjacent thereto. The cuff has two free ends 18 (only one shown) which are adapted to overlap when the cuff 14 is placed around the aorta 12.

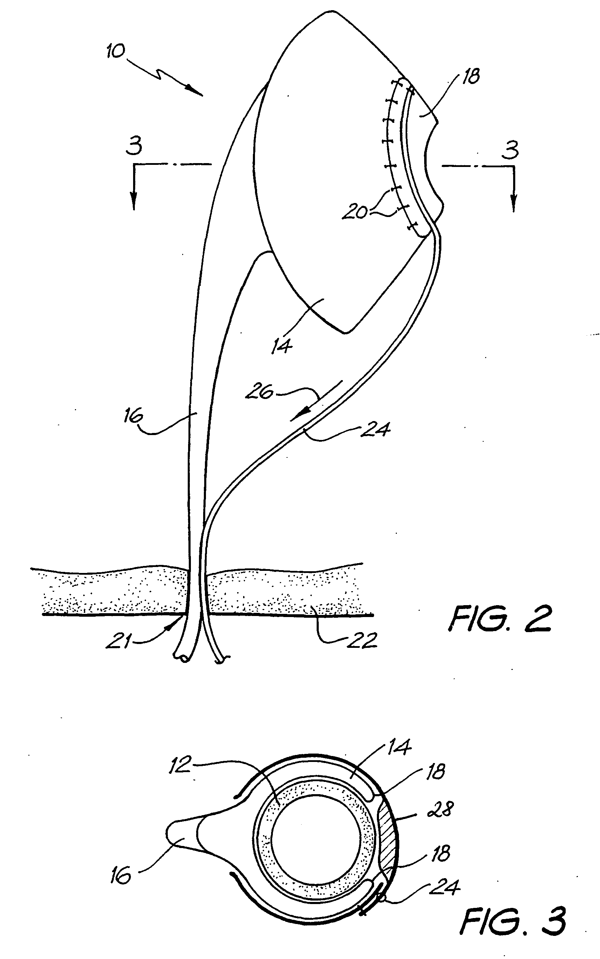

As is shown in Figs. 1 and 2, the cuff 14 is retained adjacent the aorta 12 after implantation by suturing the two free ends together at 20. A more detailed description of the cuff, its method of implantation and its method of use can be found in the applicant's above noted international PCT patent application.

As best shown in Fig. 2, the gas line 16 exits the body via a (minor) incision 21 through the skin 22 near the 5^{th} anterior intercostal space. the cuff 14 is activated (ie. inflated) and deactivated (ie. deflated) in counterpulsation with the heart by the gas line 16 being connected to an external portable IABP console, for example that known as the Datascope 97 (Datascope is a registered trade mark).

Fig. 2 also shows a release mechanism in the form of percutaneous line 24 which, when pulled from outside of the patent's body, in the direction of arrow 26, removes the sutures 20 securing the two free ends 18 of the cuff 14, thereby releasing the cuff 14 from engagement from the aorta 12. Further pulling of the line 24, again in the direction of arrow 26, withdraws the device 10 from the patient via the incision 21.

Fig. 3 shows an embodiment of a heart assist device 10 which is retained adjacent the aorta 12 by a substantially inelastic flexible sheath 28 placed around the cuff 14 and held in place by having the ends of the sheath sutured together by sutures. A more detailed description of the sheath, its method of implantation and its method of use is also found in the Applicant's above noted international PCT application, In this embodiment, the release line 24 is adapted to release connectors on two adjacent portions of the sheath 28, thereby releasing the sheath 28 from around the cuff 14 and disengaging the cuff 14 from the aorta 12. Again further pulling of the line 24 withdraws the sheath 28 and cuff 14 through a small incision in the patient. In this embodiment of the invention the connectors are separated from the sutures. A different mechanism is thus used to hold the ends of the sheath 28 together as compared with that used to release the sheath 28 from about the aorta 12.

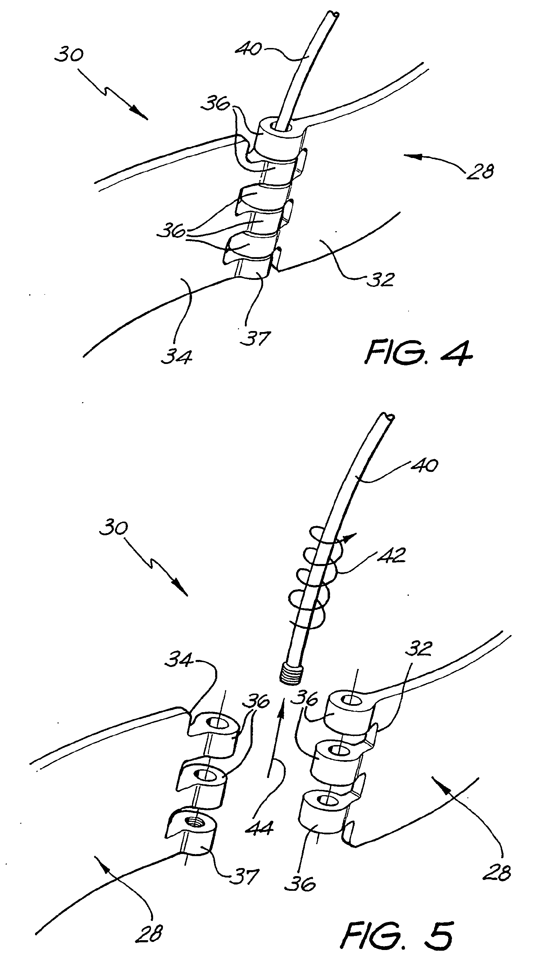

Figs. 4 and 5 show in detail a release mechanism 30 that is preferably incorporated into the sheath 28 and which is suitable for use in the embodiment of the invention shown in Fig. 3. The mechanism 30 has opposed ends 32 and 34 that are provided with complimentary spaced apart members 36 and 37. The seven uppermost member members 36 have a plain hole therein. The lower most member 37 has a threaded hole therein which is adapted to engage with a threaded grub screw 38 provided on the end of semi rigid release line 40. By being semi rigid, the release line 40 is able to bend along its longitudinal axis but not twist about its longitudinal axis when a rotational force is applied to one end. In this way, rotation of the end of the line 40 external the patient causes corresponding rotation of the other end of the line 40 containing the grub screw 38. It is to be noted that in another embodiment of the invention the mechanism 30 may be used instead of the sutures 20 and serve both the purpose of connecting the ends of the sheath 28 as well as releasing them.

In use, the sheet 30 is positioned around the cuff and the members 36 brought together to the position shown in Fig. 4. The grub screw 38 is passed through all of the members 36 and then screwed into engagement with the member 37. When the cuff 14 is to be removed, the line 40 is twisted, as indicated by arrow 42, until the grub screw 38 is free from engagement with the member 37, as indicated by arrow 44 to release the sheath 28 from around the cuff 14, and the cuff 14 from securement around the aorta 12.

Other embodiments of release mechanism (not shown) for the cuff or sheath include remotely actuated zipping mechanisms; metal wires with an end which can be heated to melt the cuff or sheath or the sutures, captive blades which may be drawn through the cuff or sheath, releaseable stitching, releaseable clips or VELCRO^{™} having a release force higher than the forces generated by inflation of the cuff but lower than the force necessary to damage to aorta.

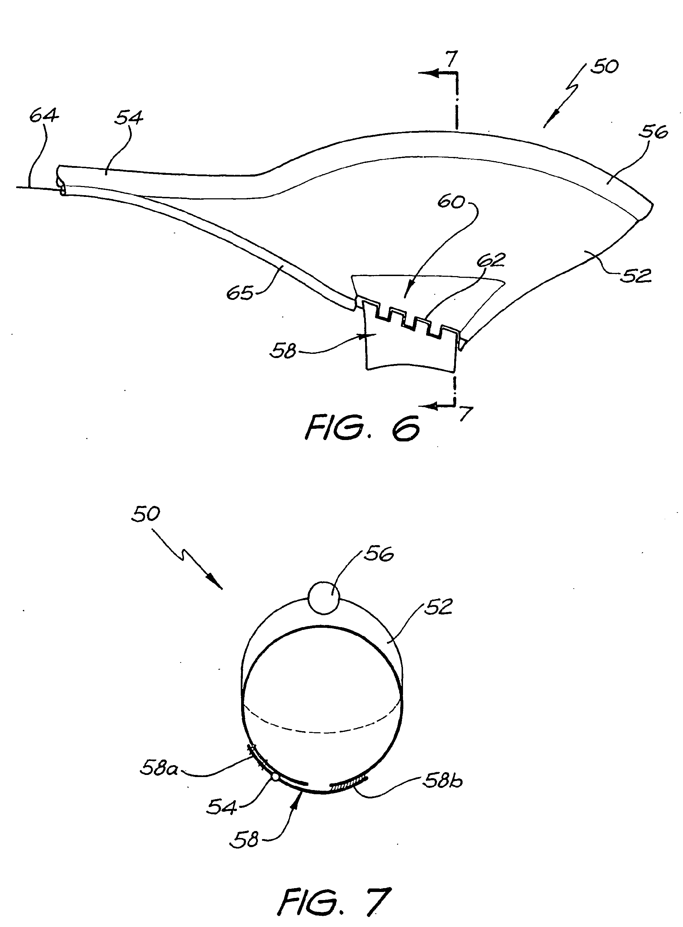

Figs 6 and 7 show a second embodiment of a heart assist device 50 in accordance with the invention. The device 50 is again suitable for implantation in the thoracic cavity of a patient adjacent the ascending portion of the aorta. The device 50 includes a hollow inflatable balloon 52 and a percutaneous gas line 54 connected to a gas duct 56. The device 50 also includes a short wrap 58 which extends across the gap between the ends of the balloon 52. One end 58a of the wrap 58 is permanently adhered to the outer surface of the balloon 52 adjacent one of its ends. The other end 58b of the wrap 58 has an adhesive patch which is caused to adhere to the other end of the balloon 52 after the device 50 is placed around the aorta of a patient. A releasable join 60 is formed intermediate the ends of the wrap 58 with a series of small open-ended, transversely extending pockets 62, which interdigitate. A wire 64 extends down a tube 65 and through the aligned and interdigitated pockets 60 to prevent the two parts of the wrap 58 from being separated. When the wire 64 is withdrawn, preferably extracorporeally in a similar manner to that described in relation to the release mechanism 30, the two parts of the wrap 58 separate allowing the device 50 to be withdrawn from the patient or, alternatively, left in the patient in an inactive state.

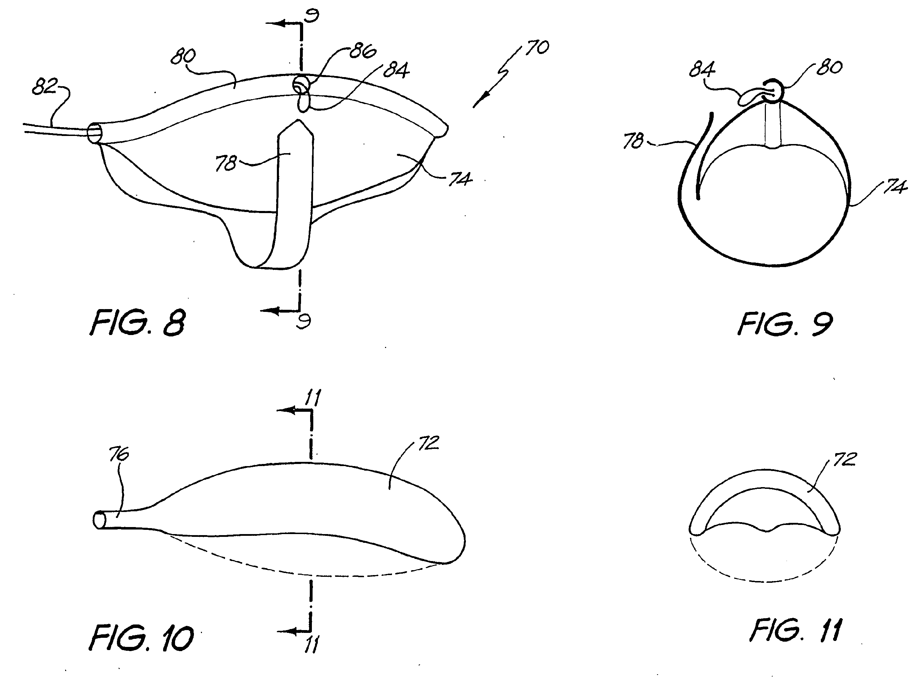

Figs 8 and 9 show a third embodiment of a heart assist device 70 in accordance with the invention. The device 70 is also suitable for implantation in the thoracic cavity of a patient adjacent to the ascending portion of the aorta. Figs 10 and 11 show, in isolation, a balloon 72 used in the device 70. A catheter 76 is connected to the balloon 72, which has a circular cross section when inflated (see solid lines) and an arcuate one when deflated (see phantom lines). A separate wrap is provided in two parts 74 and 78, which is made of a thin non-elastic synthetic plastics material. A tube 80 is positioned along the mid-line of the wrap part 78 which extends beyond the wrap part 78 and through the skin of the patient alongside the catheter. A doubled over thread 82 extends down the tube 80 and the mid-point loop 84 of the thread 82 projects through a hole 86 in the tube 80. To implant, the balloon 72 is positioned on the aorta and the wrap 74, 78 placed over it and the free end of the wrap part 78 is loosely sutured to the loop in the thread. To remove, one end of the thread 82 is withdrawn from the tube 80. This releases the free end of the wrap part 78. The balloon 72 is then inflated to urge the wrap 74, 78 off the aorta. The balloon 72 is then deflated and withdrawn. The wrap 74, 78 is then withdrawn separately.

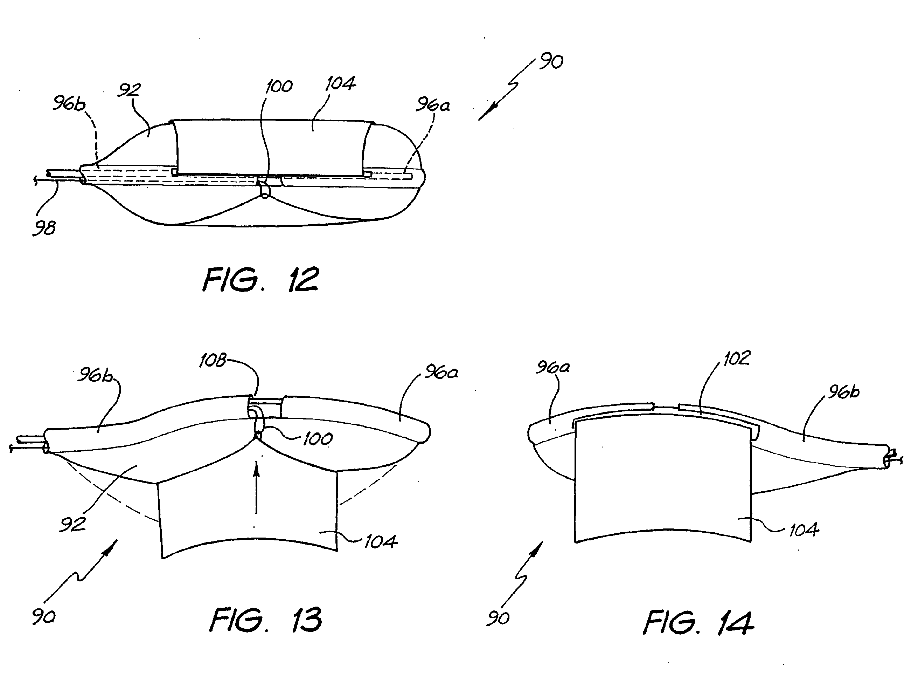

Figs 12 to 14 show a fourth embodiment of a heart assist device 90 in accordance with the invention. In this embodiment, a balloon 92 is provided with a catheter (not shown) which has a dual lumen 96a and 96b. The lumen 96a conveys gas to the balloon 92 while the other lumen 96b carries both a wire 98 and a looped thread 100. The wire 98 extends down the catheter to its proximal end and projects through a transverse open end pocket 102 in one end of a wrap 104. The wire 98 holds the wrap 104 releasively connected to the balloon 92. The thread 100 projects from an aperture 108 in the lumen 96b and is sutured to a free end of the wrap 104. To remove the device 90, the wire 98 is withdrawn which released an end of the wrap 104 from the balloon 92. The thread 100 is then pulled, which draws the wrap 104 in to the lumen 96b of the catheter. The ballooon 92 and the wrap 104 can then be withdrawn through the patient as described in relation to earlier embodiments.

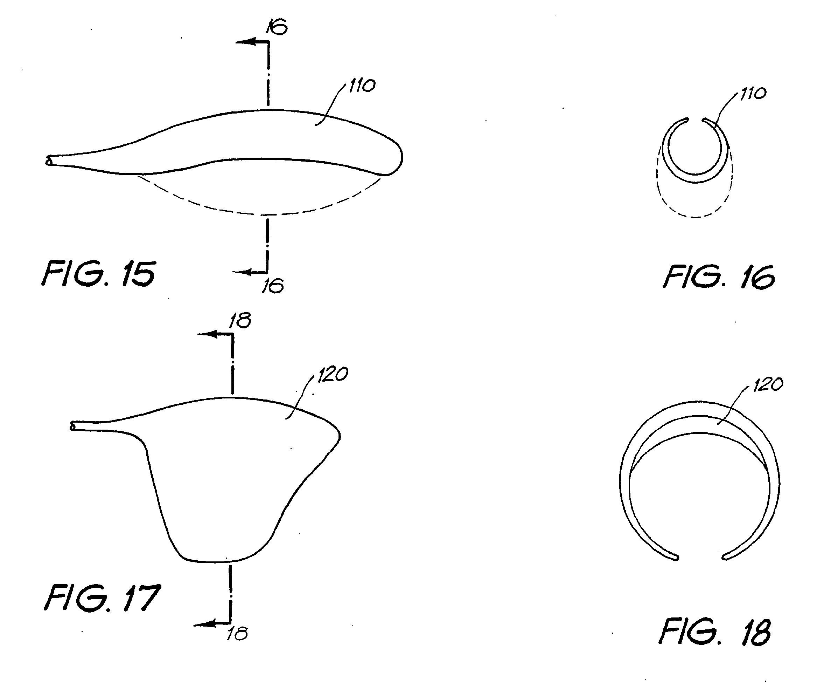

Figs 15 and 16, Figs 17 and 18, Figs 19 and 20 and Fig. 21 respectively show embodiments of balloons suitable for use in embodiments of heart assist devices according to the invention.

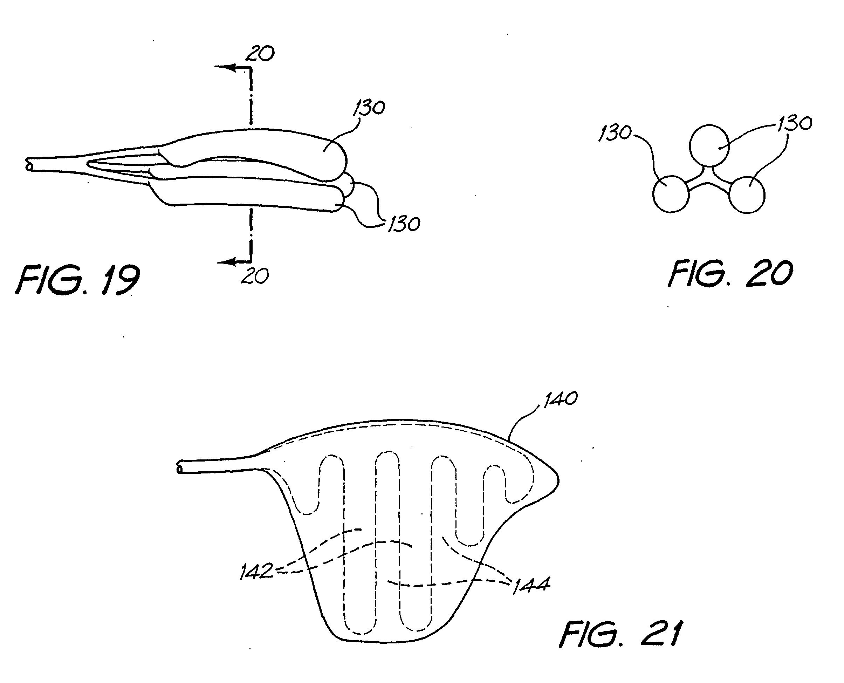

Figs 15 and 16 show an essentially cylindrical balloon 110, which is depicted deflated in solid lines and inflated in phantom lines. Figs 17 and 18 show a balloon 120 having an essentially arcuate cross section, similar to that used in the heart assist device 50. Figs 19 and 20 show an arrangement of smaller individual balloons 130 spread around the aorta and all lying in alignment with the aorta. Fig 21 shows a single balloon 140 which includes a number of smaller balloon segments 142 therein, which are separated by non-inflated portions 144. The segments 142 extend along the aorta. In a varaition of this embodiment (not shown), the segments 142 extend around the aorta.

It will be appreciated that the shape and number of balloons affects the way in which the aorta is compressed. If a single circular balloon is used then the aorta will be compressed from only one side. If the balloon encircles the aorta then the aorta will be inwardly compressed about its whole circumference. To be effective, the balloon is configured to preferably displace from 10-30ml, more preferably 15-20ml of blood from the aorta of an adult human.

The heart assist devices described above can be implanted during surgery only for this purpose. However, a particular advantage of the disclosed devices is that they can be quickly and easily temporarily installed around the patient's aorta whilst the patient is undergoing surgery for other reasons and then removed as described above without the need for any further surgery to take place. This allows two surgical operations to be avoided.

The device also allows the patient to ambulate and there is no risk of leg ischaemia. If connected to a fixed motive means (eg. the Datascope 97 IABP console) then the patient's mobility is limited by the percutaneous line. However, this limitation is overcome by use of a portable, miniaturised motive means (eg. a belt mounted, battery powered device).

The present invention is suitable for short and/or long term treatment for heart failure and/or myocardial ischaemia. The present invention can also provide a suitable bridging device for patient's awaiting heart transplantation.

It will be appreciated by persons skilled in the art that numerous variations and/or modifications may be made to the invention as shown in the specific embodiments without departing from the scope of the invention as broadly described. For example, although the invention has been described in specific reference to compression of the aorta, the device of the present invention can equally be used for the compression of the pulmonary artery to effectively act as a right ventrical assist device, and the present invention extends to this alternative aspect. The present embodiments are, therefore, to be considered in all respects as illustrative and not restrictive.

## Claims

1. An implantable device (10, 50, 70) for assisting the functioning of the heart of a patient, the device (10, 50, 70) including:
compressing means (14, 52, 72, 92, 110, 120, 130, 140) adapted to be positioned about the aorta (12) of a patient for externally engaging and compressing the aorta (12);
a flexible and non-elastic outer wrap or sheath (18, 28, 58, 74, 104) enclosing the compressing means (14, 52, 72, 92, 110, 120, 130, 140) and adapted to maintain the compressing means (14, 52, 72, 92, 110, 120, 130, 140) in engagement with the aorta (12); and
means for releasing the wrap or sheath (18, 28, 58, 74, 104) from about the aorta (12), said releasing means (24, 30) being adapted for releasing in response to intracorporeal input during minimally invasive surgery or in response to extracorporeal input,
wherein the device (10, 50, 70) is connectable to motive means adapted to activate the compressing means (14, 52, 72, 92, 110, 120, 130, 140), and the compressing means (14, 52, 72, 92, 110, 120, 130, 140), the wrap or sheath (18, 28, 58, 74, 104) and the releasing means (24, 30) are fully implantable within the thoracic cavity of the patient.

2. A device (10, 50, 70) for assisting the functioning of the heart of a patient, the device (10, 50, 70) including:
inflatable compressing means (14, 52, 72, 92, 110, 120, 130, 140) adapted to be positioned about the aorta (12) of a patient for externally engaging and compressing the aorta (12);
a flexible and non-elastic outer wrap or sheath (18, 28, 58, 74, 104) enclosing the compressing means (14, 52, 72, 92, 110, 120, 130, 140) and adapted to maintain the compressing means (14, 52, 72, 92, 110, 120, 130, 140) in engagement with the aorta (12);
means (24, 30) for releasing the wrap or sheath (18, 28, 58, 74, 104) from about the aorta (12), said releasing means (24, 30) being adapted for releasing in response to intracorporeal input during minimally invasive surgery or in response to extracorporeal input, and
motive means to periodically inflate the compressing means (14, 52, 72, 92, 110, 120, 130, 140) in counterpulsation with the rhythm of the patient's heart, the motive means being adapted for external location and connection to the compressing means (14, 52, 72, 92, 110, 120, 130, 140) via a percutaneous line (16, 56),
wherein the compressing means (14, 52, 72, 92, 110, 120, 130, 140), the wrap or sheath (18, 28, 58, 74, 104) and the releasing means (24, 30) are fully implantable within the thoracic cavity of the patient and the wrap or sheath (18, 28, 58, 74, 104) include means adapted for attachment to itself for engaging the compressing means (14, 52, 72, 92, 110, 120, 130, 140) with the aorta (12) and for detachment from itself for intracorporeal or extracorporeal releasing from the aorta (12).

3. The device (10, 50, 70) as claimed in claim 1 or 2, wherein the releasing means (24, 30) is adapted to allow minimally invasive surgical or non-surgical removal of the device (10, 50, 70) from the patient's thoracic cavity.

4. The device (10, 50, 70) as claimed in claim 1 or 2 wherein the releasing means (24, 30) is adapted to allow minimally invasive surgical or non-surgical de-activation of the device (10, 50, 70) and retention in the patient's thoracic cavity.

5. The device (10, 50, 70) as claimed in any one of the preceding claims, wherein the compressing means (14, 52, 72, 92, 110, 120, 130, 140) includes an inflatable cuff or one or more preshaped balloons for positioning against or around a portion of the aorta (12) or for wrapping around a portion of the aorta (12).

6. The device (10, 50, 70) as claimed in claim 5, wherein the balloon(s)/cuff is/are configured longitudinally to fit the curve of the ascending aorta (12).

7. The device (10, 50, 70) as claimed in claim 5 or 6, wherein the cross-section of the balloon(s)/cuff is/are C-shaped.

8. The device (10, 50, 70) as claimed in claim 5,6 or 7, wherein the balloon(s)/cuff is/are shaped such that it/they concentrically compress(es) the length of enclosed aorta (12).

9. The device (10, 50, 70) as claimed in claim 5, 6 or 7, wherein the balloon(s)/cuff is/are shaped such that it/they assymetrically compress(es) the length of enclosed aorta (12).

10. The device (10, 50, 70) as claimed in claims 5 to 9 wherein, the wrap has an elongated tongue on one side of the balloon (s)/cuff that is passed around the aorta (12) for securing by suturing, staples or the like means (20, 38) to the other side of the balloon(s)/cuff, to which the wrap is secured by sutures, staples, or like means (20, 38).

11. The device (10, 50, 70) as claimed in claims 5 to 10, wherein the balloon(s)/cuff and the wrap are a snug fit and a low profile on the aorta (12).

12. The device (10, 50, 70) as claimed in any one of claim 5 to 11, wherein the balloon(s)/cuff is/are made from a thin synthetic plastics material.

13. The device (10, 50, 70) as claimed in any one of claims 5 to 12, wherein an inner surface(es) of the balloon(s)/cuff is/are elastic and adapted to move inwardly as the balloon(s)/cuff is/are inflated.

14. The device (10, 50, 70) as claimed in any one of claims 5 to 13, wherein an outer surface(s) of the balloon(s)/cuff is/are inelastic and the wrap extends around all of the balloon (s)/cuff.

15. The device (10, 50, 70) as claimed in any one of claims 5 to 13, wherein an outer surface (s) of the balloon (s)/cuff is/are inelastic and the wrap extends only around a part of the balloon (s)/cuff.

16. The device (10, 50, 70) as claimed in claim 14 or 15, wherein outfolds or extensions of the inelastic outer surface(s) comprise the wrap or sheath (18, 28, 58, 74, 104).

17. The device (10, 50, 70) as claimed in any one of claims 12 to 16, wherein the elastic materials in which the balloon(s)/cuff is/are made include silicones.

18. The device (10, 50, 70) as claimed in any one of claims 12 to 17, wherein the relatively inelastic or inelastic plastics in which the balloon(s)/cuff is/are made include polyurethanes, copolymers of silicones and urethanes, PET and PTFE.

19. The device (10, 50, 70) as claimed in any one of claims 5 to 18, wherein the balloon(s)/cuff are connected to a catheter (16, 56, 76) which extends out of the body and which is adapted for carrying the inflating fluid into and out of the balloon(s)/cuff.

20. The device (10, 50, 70) as claimed in claim 19, wherein the fluid is a gas.

21. The device (10, 50, 70) as claimed in claim 20, wherein the gas is helium.

22. The device (10, 50, 70) as claimed in claim 19,20 or 21, wherein the catheter (16, 56, 76) is also adapted for use in withdrawing the balloon(s)/cuff from the patient and is connected to the balloon(s)/cuff sufficiently securely that the force of withdrawal will not detach the catheter (16, 56, 76) from the balloon(s)/cuff.

23. The device (10, 50, 70) as claimed in any one of claims 5 to 22, wherein the wrap or sheath (18, 28, 58, 74, 104) extends around the whole balloon(s)/cuff and is connected onto itself, or connected to the balloon(s)/cuff at each end, or extends only across the gap between the ends of the balloon(s)/cuff.

24. The device (10, 50, 70) as claimed in any one of claims 5 to 23, wherein the wrap or sheath (18, 28, 58, 74, 104) is separate from the balloon(s)/cuff.

25. The device (10, 50, 70) as claimed in any one of claims 5 to 23, wherein the wrap or sheath (18, 28, 58, 74, 104) is integral with the balloon(s)/cuff.

26. The device (10, 50, 70) as claimed in any one of claims 5 to 25, including means to releasably secure ends of the wrap or sheath (18, 28, 58, 74, 104) together.

27. The device (10, 50, 70) as claimed in claim 26, wherein the securing means is also the releasing means (24, 30).

28. The device (10, 50, 70) as claimed in claim 26, wherein the securing means is separate from the releasing means (24, 30).

29. The device (10, 50, 70) as claimed in claim 26 or 28, wherein the securing means comprises a row of suture stitches made by the surgeon when placing the device (10, 50, 70) in the patient.

30. The device (10, 50, 70) as claimed in claim 26 or 28, wherein the securing means includes: an adhesive patch on the wrap which is adapted to stick onto a corresponding part of the wrap or the balloon(s)/cuff; a sliding clasp fastener; or one or more "bundle tie" type ratchet connectors.

31. The device (10, 50, 70) as claimed in claim 26 or 28, wherein securing means includes: an end of the wrap adapted for suturing, or otherwise connecting, to a release thread extending down the catheter (16, 56, 76), which thread is connected to the balloon(s)/cuff, the two ends of the wrap, or one end of the wrap and one part of the balloon(s)/cuff, which is provided with hooks or holes through which the thread can be laced and drawn tight.

32. The device (10, 50, 70) as claimed in claims 5 to 31, wherein the releasing means (24, 30) includes: a wire which extends down the catheter and which is adapted, upon pulling, to release one end of the wrap; a thread extending down the catheter (16, 56, 76) to which one end of the wrap adapted to be connected which can be released; a thread extending down the catheter (16, 56, 76) and connected to a sliding clasp connector, which thread is adapted, upon pulling to release the sliding clasp connector; a thread extending down the catheter and connected to a knife blade positioned relative to sutures or other connecting means, which blade is adapted, upon pulling to sever and release the to sutures or other connecting means.

33. The device (10, 50, 70) as claimed in claims 5 to 31, wherein the releasing means (24, 30) includes: remotely actuated zipping mechanisms; metal wires with an end adapted to be heated to melt the balloon(s)/cuff or wrap or sutures; captive blades adapted for drawing through the balloon(s)/cuff or wrap or sutures; releaseable stitching; releaseable clips; or VELCRO^{™} having a release force higher than the forces generated by inflation of the balloon(s)/cuffbut lower than the force necessary to damage the aorta (12).

34. The device (10, 50, 70) as claimed in claims 5 to 33, wherein the releasing means (24, 30) is adapted such that, after release, the wrap is drawn into a tube adjacent to the catheter (16, 56, 76).

## Patentansprüche

1. Implantierbare Vorrichtung (10, 50, 70) zur Unterstützung der Funktion des Herzens eines Patienten, wobei die Vorrichtung (10, 50, 70) folgendes aufweist:
Kompressionsmittel (14, 52, 72, 92, 110, 120, 130, 140), welche an der Aorta (12) eines Patienten positioniert werden können um in äußerlichen Kontakt mit der Aorta (12) zu treten und Druck auf die Aorta auszuüben;
eine flexible und nicht elastische äußere Hülle oder Ummantelung (18, 28, 58, 74, 104), welche die Kompressionsmittel (14, 52, 72, 92, 110, 120, 130, 140) aufweist und dafür geeignet ist, die Kompressionsmittel (14, 52, 72, 92, 110, 120, 130, 140) in Kontakt mit der Aorta (12) zu halten; und
Mittel zum Lösen der Hülle oder der Ummantelung (18, 28, 58, 74, 104) von der Aorta (12), wobei die Mittel zum Lösen (24, 30) dafür geeignet sind, den Lösevorgang als Reaktion auf eine intrakorporale Eingabe während einer minimalinvasiven Operation oder als Antwort auf eine extrakorporale Eingabe auszuführen,
wobei die Vorrichtung (10, 50, 70) mit Aktiviermitteln verbindbar ist, welche dafür geeignet sind, die Kompressionsmittel (14, 52, 72, 92, 110, 120, 130, 140) zu aktivieren und wobei die Kompressionsmittel (14, 52, 72, 92, 110, 120, 130, 140), die Hülle oder Ummantelung (18, 28, 58, 74, 104) und die Mittel zum Lösen (24, 30) vollständig im Brustkorb eines Patienten implantierbar sind.

2. Vorrichtung (10, 50, 70) zum Unterstützen der Funktion des Herzens eines Patientens, wobei die Vorrichtung (10, 50, 70) folgende Mittel aufweist:
aufblasbare Kompressionsmittel (14, 52, 72, 92, 110, 120, 130, 140), welche geeignet sind, an der Aorta (12) eines Patientens angebracht zu werden, mit der Aorta (12) in externen Kontakt zu treten und Druck auf die Aorta (12) auszuüben;
eine flexible und nicht elastische äußere Hülle oder Ummantelung (18, 28, 58, 74, 104), welche die Kompressionsmittel (14, 52, 72, 92, 110, 120, 130, 140) aufweist und dafür geeignet ist, die Kompressionsmittel (14, 52, 72, 92, 110, 120, 130, 140) in Kontakt mit der Aorta (12) zu halten; und
Mittel zum Lösen der Hülle oder der Ummantelung (18, 28, 58, 74, 104) von der Aorta (12), wobei die Mittel zum Lösen (24, 30) dafür geeignet sind, den Lösevorgang als Reaktion auf eine intrakorporale Eingabe während einer minimalinvasiven Operation oder als Antwort auf eine extrakorporale Eingabe auszuführen, und
Aktivierungsmittel zum periodischen Aufpumpen der Kompressionsmittel (14, 52, 72, 92, 110, 120, 130, 140) in Kontrapulsation zum Rhythmus des Herzens des Patienten, wobei die Aktivierungsmittel für eine externe Anordnung geeignet sind und mit den Kompressionsmitteln (14, 52, 72, 92, 110, 120, 130, 140) mittels einer perkutanen Verbindung (16, 56) verbindbar ist,
wobei die Kompressionsmittel (14, 52, 72, 92, 110, 120, 130, 140), die Hülle oder Ummantelung (18, 28, 58, 74, 104) und die Mittel zum Lösen (24, 30) vollständig im Brustkorb eines Patienten implantierbar sind und die Hülle oder Ummantelung (18, 28, 58, 74, 104) Mittel aufweist, welche geeignet sind mit sich selbst in Verbindung zu treten um die Kompressionsmittel (14, 52, 72, 92, 110, 120, 130, 140) in Kontakt mit der Aorta (12) zu bringen und die Verbindung mit sich selbst zu lösen um die Kompressionsmittel (14, 52, 72, 92, 110, 120, 130, 140) intrakorporal oder extrakorporal von der Aorta zu lösen.

3. Vorrichtung (10, 50, 70) nach einem der Ansprüche 1 oder 2,
**dadurch gekennzeichnet, dass** die Mittel zum Lösen (24, 30) dafür geeignet sind, eine mimalinvasive, operative oder nicht operative Entfernung der Vorrichtung (10, 50, 70) aus dem Brustraum des Patienten zu ermöglichen.

4. Vorrichtung (10, 50, 70) nach einem der Ansprüche 1 oder 2,
**dadurch gekennzeichnet, dass** die Mittel zum Lösen (24, 30) dafür geeignet sind, eine Deaktivierung der Vorrichtung (10, 50, 70) und den Verbleib im Brustraum des Patientens mittels minimalinvasiver, operativer oder nicht operativer Methoden zu ermöglichen.

5. Vorrichtung (10, 50, 70) nach einem der vorangehenden Ansprüche,
**dadurch gekennzeichnet, dass** die Kompressionsmittel (14, 52, 72, 92, 110, 120, 130, 140) einen aufblasbaren Kragen oder einen oder mehrere vorgefertigte Ballone aufweisen, welche gegen oder um einen Teil der Aorta (12) platziert oder herumgewickelt werden können.

6. Vorrichtung (10, 50, 70) nach Anspruch 5,
**dadurch gekennzeichnet, dass** der/die Ballon(e)/ Kragen longitudinal an die Kurve der aufsteigenden Aorta (12) angepasst sind/ist.

7. Vorrichtung (10, 50, 70) nach einem der Ansprüche 5 oder 6,
**dadurch gekennzeichnet, dass** der Querschnitt des/der Ballon(e)/ Kragens C-förmig ist.

8. Vorrichtung (10, 50, 70) nach einem der Ansprüche 5, 6 oder 7,
**dadurch gekennzeichnet, dass** der/die Ballon(e)/ Kragen derart geformt ist/sind, dass er/sie konzentrischen Druck auf die Länge der Aorta (12) ausüben.

9. Vorrichtung (10, 50, 70) nach einem der Ansprüche 5, 6 oder 7,
**dadurch gekennzeichnet, dass** der/die Ballon(e)/ Kragen derart geformt ist/sind, dass er/sie asymmetrischen Druck auf die Länge der Aorta (12) ausüben.

10. Vorrichtung (10, 50, 70) nach einem der Ansprüche 5 bis 9,
**dadurch gekennzeichnet, dass** die Hülle eine längliche Zunge an der einen Seite des/der Ballon(e)/ Kragens aufweist, welche um die Aorta (12) geschlungen ist, um an der anderen Seite des/der Ballon(e)/des Kragens durch Nähte und Tacker oder ähnliche Mittel (20, 38) gesichert zu werden, an welcher die Hülle durch Nähte und Tacker oder ähnliche Mittel (20, 38) gesichert ist.

11. Vorrichtung (10, 50, 70) nach einem der Ansprüche 5 bis 10,
**dadurch gekennzeichnet, dass** der/die Ballon(e)/ Kragen und die Hülle an die Kontur der Aorta (12) angepasst ist/sind.

12. Vorrichtung (10, 50, 70) nach einem der Ansprüche 5 bis 11,
**dadurch gekennzeichnet, dass** der/die Ballon(e)/ Kragen aus synthetischem Kunststoffmaterial hergestellt sind/ist.

13. Vorrichtung (10, 50, 70) nach einem der Ansprüche 5 bis 12,
**dadurch gekennzeichnet, dass** die inneren Oberflächen des/der Ballon(e)/ Kragens elastisch ist/sind und geeignet ist/sind, sich bei dem Aufpumpvorgang des/der Ballon(e)/ Kragens nach innen zu bewegen.

14. Vorrichtung (10, 50, 70) nach einem der Ansprüche 5 bis 13,
**dadurch gekennzeichnet, dass** eine der äußeren Oberflächen des/der Ballon(e)/ Kragens unelastisch ist/sind und die Hülle den/die gesamten Ballon(e)/ Kragen umgibt.

15. Vorrichtung (10, 50, 70) nach einem der Ansprüche 5 bis 13,
**dadurch gekennzeichnet, dass** eine der äußeren Oberflächen des/der Ballon(e)/ Kragens unelastisch ist/sind und die Hülle nur einen Teil des/der Ballon(e)/ Kragen umgibt.

16. Vorrichtung (10, 50, 70) nach einem der Ansprüche 14 oder 15,
**dadurch gekennzeichnet, dass** die Erstreckungen oder Auswölbungen der unelastischen äußeren Oberfläche die Hülle oder die Ummantelung (18, 28, 58, 74, 104) enthalten.

17. Vorrichtung (10, 50, 70) nach einem der Ansprüche 12 bis 16,
**dadurch gekennzeichnet, dass** die elastischen Materialien des/der Ballon(e)/ Kragens Silikone aufweisen.

18. Vorrichtung (10, 50, 70) nach einem der Ansprüche 12 bis 17,
**dadurch gekennzeichnet, dass** die relativ unelastischen oder unelastischen Kunststoffe, aus welchem der/die Ballon(e)/ Kragen hergestellt ist/sind, Polyruthane, Copolymere von Silikonen und Urethanen, PET und PTFE aufweisen.

19. Vorrichtung (10, 50, 70) nach einem der Ansprüche 5 bis 18,
**dadurch gekennzeichnet, dass** der/die Ballon(e)/ Kragen mit einem Katheter (16, 56, 76) verbunden ist/sind, welcher sich aus dem Körper heraus erstreckt und geeignet ist ein Pumpfluid in den/die Ballone/ Kragen hinein- und herauszuleiten.

20. Vorrichtung (10, 50, 70) nach Anspruch 19,
**dadurch gekennzeichnet, dass** Fluid ein Gas ist.

21. Vorrichtung (10, 50, 70) nach Anspruch 20,
**dadurch gekennzeichnet, dass** das Gas Helium ist.

22. Vorrichtung (10, 50, 70) nach einem der Ansprüche 19, 20 oder 21,
**dadurch gekennzeichnet, dass** der Katheter (16, 56, 76) ebenfalls dafür geeignet ist den/die Ballon(e)/ Kragen aus dem Patienten zu entfernen und mit dem/den Ballon(en)/ Kragen ausreichend sicher verbunden ist/sind, sodass die zum Entfernen notwendige Kraft den Katheter (16, 56, 76) nicht von dem/den Ballon(en)/dem Kragen löst.

23. Vorrichtung (10, 50, 70) nach einem der Ansprüche 5 bis 22,
**dadurch gekennzeichnet, dass** sich die Hülle oder Ummantelung (18, 28, 58, 74, 104) um den/die gesamten Ballon(e)/ Kragen herum erstreckt und mit sich selbst verbunden ist und mit jedem Ende des/der Ballon(e)/ Kragens verbunden ist oder sich nur in dem Spalt zwischen den Enden des/der Ballon(e)/ Kragens erstreckt.

24. Vorrichtung (10, 50, 70) nach einem der Ansprüche 5 bis 23,
**dadurch gekennzeichnet, dass** die Hülle oder Ummantelung (18, 28, 58, 74, 104) ein von dem/den Ballonen/ Kragen separates Teil ist.

25. Vorrichtung (10, 50, 70) nach einem der Ansprüche 5 bis 23,
**dadurch gekennzeichnet, dass** die Hülle oder Ummantelung (18, 28, 58, 74, 104) integral mit dem/den Ballon(en)/ Kragen ausgestaltet ist.

26. Vorrichtung (10, 50, 70) nach einem der Ansprüche 5 bis 25,
**dadurch gekennzeichnet, dass** Mittel zum reversiblen Sicheren der Enden der Hülle oder Ummantelung (18, 28, 58, 74, 104) miteinander vorgesehen sind.

27. Vorrichtung (10, 50, 70) nach Anspruch 26,
**dadurch gekennzeichnet, dass** die Mittel zum Sichern identisch sind mit den Mitteln zum Lösen (24, 30).

28. Vorrichtung (10, 50, 70) nach Anspruch 26,
**dadurch gekennzeichnet, dass** die Mittel zum Sichern unterschiedlich sind von den Mitteln zum Lösen (24, 30).

29. Vorrichtung (10, 50, 70) nach einem der Ansprüche 26 oder 28,
**dadurch gekennzeichnet, dass** die Mittel zum Sichern eine Reihe von genähten Stichen aufweisen, welche durch einen Chirurg während des Platzierens der Vorrichtung (10, 50, 70) im Patienten gesetzt worden sind.

30. Vorrichtung (10, 50, 70) nach einem der Ansprüche 26 oder 28,
**dadurch gekennzeichnet, dass** die Mittel zum Sichern beinhalten:
eine Klebestelle auf der Hülle aufweisen, welche dafür geeignet sind, auf einem korrespondierenden Teil auf der Hülle oder des/den Ballon(s)/ Kragens zu kleben; oder ein Schiebeverschluss; oder eine oder mehrere bündelartige Klinkenverbindungen vorgesehen sind.

31. Vorrichtung (10, 50, 70) nach einem der Ansprüche 26 oder 28,
**dadurch gekennzeichnet, dass** die Mittel zum Sichern des Weiteren beinhalten:
ein Ende der Hülle oder Ummantelung geeignet zum Nähen, oder anderweitiger Verbindung zu einem Lösefaden, welcher sich durch den Katheter (16, 56, 76) nach unten erstreckt, wobei der Faden mit dem/den Ballon(en)/Kragen verbunden ist und die beiden Enden der Hülle oder ein Ende der Hülle und ein Teil des/der Ballon(e)/ Kragens mit Haken oder Löchern ausgestattet ist, durch welche der Faden hindurch geführt und strammgezogen werden kann.

32. Vorrichtung (10, 50, 70) nach einem der Ansprüche 5 bis 31,
**dadurch gekennzeichnet, dass** die Mittel zum Lösen (24, 30) folgendes beinhalten:
ein Draht, welcher sich durch den Katheter erstreckt und dafür geeignet ist durch Zug ein Ende der Hülle zu lösen;
ein Faden, welcher sich durch den Katheter (16, 56, 76) erstreckt und welchen ein Ende der Hülle befestigbar ist, so dass es gelöst werden kann;
ein Faden, welcher sich durch den Katheter (16, 56, 76) erstreckt und mit einer Schiebeschließe verbunden ist, wobei der Faden dafür geeignet ist durch Zug die Schiebeschließe zu lösen;
ein Faden, welcher sich durch den Katheter nach unten erstreckt und mit einer Messerklinge verbunden ist, welche in relativer Nähe zu Nähten oder anderen Verbindungsmittel positioniert ist, wobei die Schneide dafür geeignet ist durch Zug die Nähte oder andere Verbindungsmittel zu zerschneiden und zu lösen.

33. Vorrichtung (10, 50, 70) nach einem der Ansprüche 5 bis 31,
**dadurch gekennzeichnet, dass** die Mittel zum Lösen (24, 30) beinhalten:
einen fernbedienbaren Reißverschlussmechanismus;
metallene Drähte, wobei ein Ende der Drähte dafür geeignet ist erhitzt zu werden und den/die Ballon(e)/ Kragen oder die Hülle oder die Nähte zu schmelzen;
feste Schneiden, welche geeignet sind durch den/die Ballon(e)/ Kragen oder die Hülle oder die Nähte zu schneiden;
lösbare Nähte;
lösbare Clips;
oder VELCRO^{™} mit einer Kraft zum Lösen, welche höher ist als die Kraft, welche durch das Aufpumpen des/der Ballon(e)/ Kragens generiert wird und kleiner ist als die Kraft, welche notwendig ist um die Aorta (12) zu beschädigen.

34. Vorrichtung (10, 50, 70) nach einem der Ansprüche 5 bis 33,
**dadurch gekennzeichnet, dass** die Mittel zum Lösen (24, 30) dafür geeignet sind, nach dem Lösen die Hülle in eine Röhre neben dem Katheter (16, 56, 76) zu ziehen.

## Revendications

1. Dispositif implantable (10, 50, 70) pour aider le fonctionnement du coeur d'un patient, le dispositif (10, 50, 70) comprenant :
des moyens de compression (14, 52, 72, 92, 110, 120, 130, 140) adaptés pour être positionnés autour de l'aorte (12) d'un patient pour mettre en prise et compresser extérieurement l'aorte (12) ;
une enveloppe ou gaine externe flexible et non élastique (18, 28, 58, 74, 104) enfermant les moyens de compression (14, 52, 72, 92, 110, 120, 130, 140) et adaptée pour maintenir les moyens de compression (14, 52, 72, 92, 110, 120, 130, 140) en mise en prise avec l'aorte (12) ; et
des moyens pour libérer l'enveloppe ou gaine (18, 28, 58, 74, 104) de l'aorte (12), lesdits moyens de libération (24, 30) étant adaptés pour libérer, en réponse à une entrée intracorporelle pendant la chirurgie minimalement invasive ou en réponse à une entrée extracorporelle,
dans lequel le dispositif (10, 50, 70) peut être raccordé à des moyens moteurs adaptés pour activer les moyens de compression (14, 52, 72, 92, 110, 120, 130, 140) et les moyens de compression (14, 52, 72, 92, 110, 120, 130, 140), l'enveloppe ou gaine (18, 28, 58, 74, 104) et les moyens de libération (24, 30) sont complètement implantables à l'intérieur de la cavité thoracique du patient.

2. Dispositif (10, 50, 70) pour aider le fonctionnement du coeur d'un patient, le dispositif (10, 50, 70) comprenant :
les moyens de compression gonflables (14, 52, 72, 92, 110, 120, 130, 140) adaptés pour être positionnés autour de l'aorte (12) d'un patient pour mettre en prise et comprimer extérieurement l'aorte (12) ;
une enveloppe ou gaine externe flexible et non élastique (18, 28, 58, 74, 104) enfermant les moyens de compression (14, 52, 72, 92, 110, 120, 130, 140) et adaptée pour maintenir les moyens de compression (14, 52, 72, 92, 110, 120, 130, 140) en mise en prise avec l'aorte (12) ;
des moyens (24, 30) pour libérer l'enveloppe ou gaine (18, 28, 58, 74, 104) de l'aorte (12), lesdits moyens de libération (24, 30) étant adaptés pour libérer en réponse à une entrée intracorporelle pendant une chirurgie minimalement invasive ou en réponse à une entrée extracorporelle, et
des moyens moteurs pour gonfler périodiquement les moyens de compression (14, 52, 72, 92, 110, 120, 130, 140) en contrepulsation avec le rythme du coeur du patient, et les moyens moteurs étant adaptés pour l'emplacement et le raccordement externe aux moyens de compression (14, 52, 72, 92, 110, 120, 130, 140) via une conduite percutanée (16, 56),
dans lequel les moyens de compression (14, 52, 72, 92, 110, 120, 130, 140), l'enveloppe ou gaine (18, 28, 58, 74, 104) et les moyens de libération (24, 30) sont complètement implantables à l'intérieur de la cavité thoracique du patient et l'enveloppe ou gaine (18, 28, 58, 74, 104) comprennent des moyens adaptés pour la fixation sur elle-même afin de mettre en prise les moyens de compression (14, 52, 72, 92, 110, 120, 130, 140) avec l'aorte (12) et pour le détachement d'elle-même pour la libération intracorporelle ou extracorporelle de l'aorte (12).

3. Dispositif (10, 50, 70) selon la revendication 1 ou 2, dans lequel les moyens de libération (24, 30) sont adaptés pour permettre le retrait non chirurgical ou chirurgical minimalement invasif du dispositif (10, 50, 70) de la cavité thoracique du patient.

4. Dispositif (10, 50, 70) selon la revendication 1 ou 2, dans lequel les moyens de libération (24, 30) sont adaptés pour permettre la désactivation non chirurgicale ou chirurgicale minimalement invasive du dispositif (10, 50, 70) et la rétention dans la cavité thoracique du patient.

5. Dispositif (10, 50, 70) selon l'une quelconque des revendications précédentes, dans lequel les moyens de compression (14, 52, 72, 92, 110, 120, 130, 140) comprennent une manchette gonflable ou un ou plusieurs ballonnets préformés pour le positionnement contre ou autour d'une partie de l'aorte (12) ou pour l'enveloppement autour d'une partie de l'aorte (12).

6. Dispositif (10, 50, 70) selon la revendication 5,
dans lequel le(les) ballonnet(s)/manchette est/sont configuré(s) longitudinalement pour s'adapter à la courbure de l'aorte ascendante (12).

7. Dispositif (10, 50, 70) selon la revendication 5 ou 6, dans lequel la section transversale du(des) ballonnet(s)/manchette est en forme de C.

8. Dispositif (10, 50, 70) selon la revendication 5, 6 ou 7, dans lequel le(les) ballonnet(s)/manchette est/sont formé(s) de sorte qu'il(elle)/ils comprime(ent) de manière concentrique la longueur de l'aorte (12) enfermée.

9. Dispositif (10, 50, 70) selon la revendication 5, 6 ou 7, dans lequel le(les) ballonnet(s)/manchette est/sont formé(s) de sorte qu'il(elle)/ils comprime(ent) de manière asymétrique la longueur de l'aorte (12) enfermée.

10. Dispositif (10, 50, 70) selon les revendications 5 à 9, dans lequel l'enveloppe a une languette allongée d'un côté du(des) ballonnet(s)/manchette qui passe autour de l'aorte (12) pour la fixation par suture, agrafes, ou des moyens similaires (20, 38) sur l'autre côté du(des) ballonnet(s)/manchette, auxquels l'enveloppe est fixée par suture, agrafes, ou moyens similaires (20, 38).

11. Dispositif (10, 50, 70) selon les revendications 5 à 10, dans lequel le(les) ballonnet(s)/manchette et l'enveloppe a(ont) un ajustement serré et un profil bas sur l'aorte (12).

12. Dispositif (10, 50, 70) selon l'une quelconque des revendications 5 à 11, dans lequel le(les) ballonnet(s)/manchette est/sont réalisé(s) à partir d'une matière plastique synthétique fine.

13. Dispositif (10, 50, 70) selon l'une quelconque des revendications 5 à 12, dans lequel une(des) surface(s) interne(s) du(des) ballonnet(s)/manchette est/sont élastique(s) et adaptée(s) pour se déplacer vers l'intérieur lorsque le(les) ballonnet(s)/manchette est/sont gonflé(s).

14. Dispositif (10, 50, 70) selon l'une quelconque des revendications 5 à 13, dans lequel une(des) surface(s) externe(s) du(des) ballonnet(s)/manchette est/sont inélastique(s) et l'enveloppe s'étend autour de tout le(les) ballonnet(s)/manchette.

15. Dispositif (10, 50, 70) selon l'une quelconque des revendications 5 à 13, dans lequel une(des) surface(s) externe(s) du(des) ballonnet(s)/manchette est/sont inélastique(s) et l'enveloppe s'étend uniquement autour d'une partie du(des) ballonnet(s)/manchette.

16. Dispositif (10, 50, 70) selon la revendication 14 ou 15, dans lequel des replis ou extensions de l'une (des) une(des) surface(s) externe(s) inélastique(s) comprennent l'enveloppe ou la gaine (18, 28, 58, 74, 104).

17. Dispositif (10, 50, 70) selon l'une quelconque des revendications 12 à 16, dans lequel les matériaux élastiques avec lesquels le(les) ballonnet(s)/manchette est/sont réalisé(s) comprennent des silicones.

18. Dispositif (10, 50, 70) selon l'une quelconque des revendications 12 à 17, dans lequel la matière plastique relativement inélastique ou inélastique avec laquelle le(les) ballonnet(s)/manchette est/sont fabriqué(s), comprend les polyuréthanes, les copolymères de silicones et d'uréthanes, le PET et le PTFE.

19. Dispositif (10, 50, 70) selon l'une quelconque des revendications 5 à 18, dans lequel le(les) ballonnet(s)/manchette est/sont raccordé(s) à un cathéter (16, 56, 76) qui s'étend à l'extérieur du corps et qui est adapté pour transporter le fluide de gonflage à l'intérieur et à l'extérieur du(des) ballonnet(s)/manchette.

20. Dispositif (10, 50, 70) selon la revendication 19,
dans lequel le fluide est un gaz.

21. Dispositif (10, 50, 70) selon la revendication 20,
dans lequel le gaz est de l'hélium.

22. Dispositif (10, 50, 70) selon la revendication 19, 20 ou 21, dans lequel le cathéter (16, 56, 76) est également adapté pour être utilisé pour retirer le (les) ballonnet(s)/manchette du patient et est raccordé au(aux) ballonnet(s)/manchette de manière suffisamment sûr que la force de retrait ne détache pas le cathéter (16, 56, 76) du(des) ballonnet(s)/manchette.

23. Dispositif (10, 50, 70) selon l'une quelconque des revendications 5 à 22, dans lequel l'enveloppe ou la gaine (18, 28, 58, 74, 104) s'étend autour de tout le (les) ballonnet(s)/manchette et est raccordée sur elle-même ou raccordée au(aux) ballonnet(s)/manchette au niveau d'une extrémité, ou s'étend uniquement sur l'espace entre les extrémités du(des) ballonnet(s)/manchette.

24. Dispositif (10, 50, 70) selon l'une quelconque des revendications 5 à 23, dans lequel l'enveloppe ou la gaine (18, 28, 58, 74, 104) est séparée du(des) ballonnet(s)/manchette.

25. Dispositif (10, 50, 70) selon l'une quelconque des revendications 5 à 23, dans lequel l'enveloppe ou la gaine (18, 28, 58, 74, 104) est solidaire du(des) ballonnet(s)/manchette.

26. Dispositif (10, 50, 70) selon l'une quelconque des revendications 5 à 25, comprenant des moyens pour fixer de manière amovible les extrémités de l'enveloppe ou gaine (18, 28, 58, 74, 104) ensemble.

27. Dispositif (10, 50, 70) selon la revendication 26,
dans lequel les moyens de fixation sont également les moyens de libération (24, 30).

28. Dispositif (10, 50, 70) selon la revendication 26,
dans lequel les moyens de fixation sont séparés des moyens de libération (24, 30).

29. Dispositif (10, 50, 70) selon la revendication 26 ou 28, dans lequel les moyens de fixation comprennent une rangée de points de suture réalisés par le chirurgien lorsqu'il place le dispositif (10, 50, 70) dans le patient.

30. Dispositif (10, 50, 70) selon la revendication 26 ou 28, dans lequel les moyens de fixation comprennent : une pièce adhésive sur l'enveloppe qui est adaptée pour se coller sur une partie correspondante de l'enveloppe ou du(des) ballonnet(s)/manchette ; une fixation à crochet coulissant ; ou un ou plusieurs connecteurs à encliquetage de type « ficeleuse »

31. Dispositif (10, 50, 70) selon la revendication 26 ou 28, dans lequel les moyens de fixation comprennent : une extrémité de l'enveloppe adaptée pour la suture ou bien le raccordement à un fil de libération s'étendant du cathéter (16, 56, 76), lequel fil est raccordé au (aux) ballonnet(s)/manchette, aux deux extrémités de l'enveloppe ou à une extrémité de l'enveloppe, et une partie du(des) ballonnet(s)/manchette qui est prévue avec des crochets ou des trous à travers lesquels le fil peut être lacé et serré.

32. Dispositif (10, 50, 70) selon les revendications 5 à 31, dans lequel les moyens de libération (24, 30) comprennent :
un fil qui s'étend vers le bas du cathéter (16, 56, 76) et qui est adapté, après la traction, pour libérer une extrémité de l'enveloppe ; un fil s'étendant vers le bas du cathéter (16, 56, 76) auquel une extrémité de l'enveloppe adaptée pour être raccordée qui peut être libérée ; un fil s'étendant vers le bas du cathéter (16, 56, 76) est raccordé à un connecteur de crochet coulissant, lequel fil est adapté, suit à la traction, à libérer le connecteur à crochet coulissant ; un fil s'étendant vers le bas du cathéter (16, 56, 76) et raccordé à une lame positionnée par rapport aux sutures ou d'autres moyens de raccordement, laquelle lame est adaptée, suite à la traction, à couper et libérer les sutures ou d'autres moyens de raccordement.

33. Dispositif (10, 50, 70) selon les revendications 5 à 31, dans lequel les moyens de libération (24, 30) comprennent :
des mécanismes de fermeture éclaire actionnés à distance; des fils métalliques avec une extrémité adaptée pour être chauffés afin de faire fondre le(les) ballonnet(s)/manchette ou enveloppe ou sutures ; des lames captives adaptées pour tirer sur le(les) ballonnet(s)/manchette ou enveloppe ou sutures ; des points amovibles ; des agrafes amovibles ; ou du VELCRO^{™} ayant une force de libération supérieure aux forces générées par le gonflage du(des) ballonnet(s)/manchette mais inférieure à la force nécessaire pour endommager l'aorte (12).

34. Dispositif (10, 50, 70) selon les revendications 5 à 33, dans lequel les moyens de libération (24, 30) sont adaptés de telle sorte que, après la libération, l'enveloppe est retiré dans un tube adjacent au cathéter (16, 56, 76).
